(19) Europäisches Patentamt
European Patent Office
Office européen des brevets

(11) Veröffentlichungsnummer: **0 003 061**
**B1**

(12) **EUROPÄISCHE PATENTSCHRIFT**

(45) Veröffentlichungstag der Patentschrift: **20.05.81**

(51) Int. Cl.³: **C 07 D 251/10**, A 01 N 43/64

(21) Anmeldenummer: **78101876.7**

(22) Anmeldetag: **29.12.78**

(54) **(1,2,3,4,5,6H)-Triazin-2.4-dione, Verfahren zu ihrer Herstellung sowie ihre Verwendung als Insektizide.**

(30) Priorität: **11.01.78 DE 2801029**

(43) Veröffentlichungstag der Anmeldung: **25.07.79 Patentblatt 79/15**

(45) Bekanntmachung des Hinweises auf die Patenterteilung: **20.05.81 Patentblatt 81/20**

(84) Benannte Vertragsstaaten: **BE CH DE FR GB IT NL**

(56) Entgegenhaltungen:
**DE-A-1 670 668**
**DE-A-2 460 824**
**FR-A-2 317 283**
**BULLETIN DE LA SOCIETE CHIMIQUE DE FRANCE, nr. 5—6 (1975) Paris, FR.**
**A. ETIENNE et al. »Sur quelques dioxo-2,4-triazines-1,3,5« Seiten 1419—1424**

(73) Patentinhaber: **BAYER AG, Zentralbereich Patente, Marken und Lizenzen, D-5090 Leverkusen 1, Bayerwerk (DE)**

(72) Erfinder: **Möhring, Edgar, Dr., Hufer Weg 49a, D-5060 Bergisch-Gladbach 2 (DE)**
Erfinder: **Stadler, Peter, Dr., Ohligser Strasse 85, D-5657 Haan (DE)**
Erfinder: **Roessler, Peter, Dr., Elster Strasse 15, D-5060 Bergisch-Gladbach 1 (DE)**

**EP 0 003 061 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

BUNDESDRUCKEREI BERLIN

(1,2,3,4,5,6H)-Triazin-2,4-dione, Verfahren zu ihrer Herstellung sowie ihre Verwendung als Insektizide

Die vorliegende Erfindung betrifft neue (1,2,3,4,5,5H)-Triazin-2,4-dione, Verfahren zu ihrer Herstellung sowie ihre Verwendung als Insektizide.

Es ist bereits bekannt, daß Triazin-2,4-dione gute insektizide Wirksamkeit besitzen. Die Wirkung dieser Verbindungen befriedigt jedoch nicht in allen Fällen (vgl. Deutsche Offenlegungsschrift 2 543 497).

Weiterhin sind aus Bulletin de Societé Chimique de France, Nr. 5–6 (1975), Seiten 1419–1424, 1,3-Dimethyl-6-phenyl(und 2,6-dichlorphenyl)-(1,2,3,4,56H)-triazin-2,4-dion bekannt, also Verbindungen, welche in 6-Stellung einen Arylrest enthalten. In dieser Literaturstelle wird sehr allgemein auf die Möglichkeit einer Wirkung der Verbindungen auf dem Pflanzenschutzgebiet hingewiesen, wobei jedoch nicht zu erkennen ist, an welchen der vielen Bereiche des Pflanzenschutzes die Autoren gedacht hatten.

Es wurden nun (1,2,3,4,5,6H)-Triazin-2,4-dione der allgemeinen Formel (I)

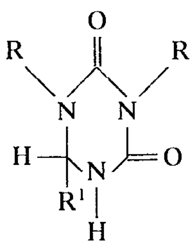

(I)

gefunden, in welcher

R für gegebenenfalls ein- oder mehrfach durch Halogen, Cyano, Aryloxy, Alkoxy, Halogenalkoxy substituiertes geradkettiges, verzweigtes oder cyclisches Alkyl mit bis zu 20 C-Atomen oder für Phenyl oder Naphthyl steht, die gegebenenfalls ein- oder mehrfach durch Halogen, CN, Alkyl $C_{1-4}$, Mono- oder Dialkylamino, Halogenalkyl, gegebenenfalls durch Halogen und/oder Halogenalkyl substituiertes Phenoxy, Alkoxy, Halogenalkoxy substituiert sind weiterhin für gegebenenfalls durch Halogen, Cyano, Mono- oder Dialkylamino, Alkoxy substituiertes Phenylsulfonyl oder für gegebenenfalls durch Halogen oder Cyano substituiertes Alkylsulfonyl, steht, und

$R^1$ für Wasserstoff, für geradkettiges verzweigtes oder cyclisches Alkyl mit bis zu 10 C-Atomen, das gegebenenfalls substituiert ist durch Halogen, Alkoxy oder Cyano, steht.

Diese Verbindungen werden erhalten, indem man Triazin-2,4-dione der allgemeinen Formel (II)

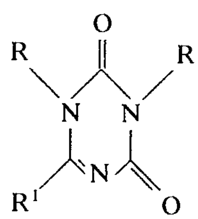

(II)

in welcher
R und $R^1$ die oben angegebene Bedeutung haben,
katalytisch in an sich bekannter Weise hydriert.

Es war auch bei Kenntnis des Standes der Technik überraschend und nicht vorherzusehen, daß die erfindungsgemäßen hydrierten Triazin-2,4-dione ausgezeichnete, die Entwicklung von Insekten hemmende Wirkung aufweisen.

Verwendet man 1,3-Diphenyl-5-azauracil als Ausgangsverbindung, kann der Reaktionsablauf durch das folgende Formelschema wiedergegeben werden:

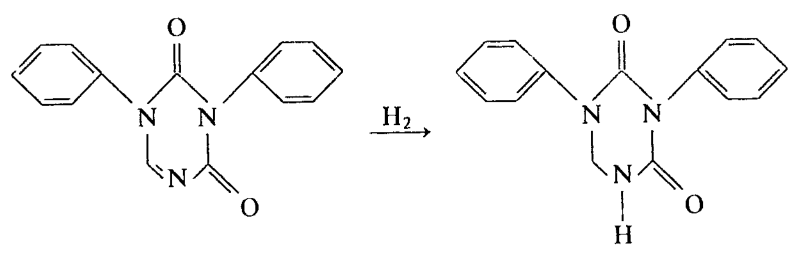

Die erfindungsgemäßen neuen (1,2,3,4,5,6H)-Triazin-2,4-dione sind durch die allgemeine Formel (I) definiert. In dieser Formel steht $R^1$ bevorzugt für Wasserstoff, Methyl, Äthyl, n-Propyl, Isopropyl, n-Butyl, Chlorhexyl, Phenyl. In dieser Formel steht R bevorzugt für Methyl, Äthyl, n-Propyl, n-Butyl, Chlorhexyl, Phenyl, 4-Chlorphenyl, 3,4-Dichlorphenyl, 4-Methoxyphenyl, 2-Trifluormethylphenyl, 3-Trifluormethylphenyl, 3-Chlor-4-Trifluormethylphenyl, 4-Trifluormethoxyphenyl, 4-p-Phenoxyphenyl, 4-(3'-Trifluormethyl)-phenoxyphenyl, 4-Fluorphenyl, 4-Trifluormethylphenyl, 4-Chlor-3-trifluormethylphenyl.

Besonders zu erwähnen sind folgende der erfindungsgemäßen Verbindungen:

1,3-Di-p-chlorphenyl-(1H,2H,3H,4H,5H,6H)-triazin-2,4-dion,
1,3-Dimethyl-(1H,2H,3H,4H,5H,6H)-triazin-2,4-dion,
1,3-Diisopropyl-(1H,2H,3H,4H,5H,6H)-triazin-2,4-dion,
1,3-Di-p-fluorphenyl-(1H,2H,3H,4H,5H,6H)-triazin-2,4-dion,
1,3-Bis-4-trifluormethylphenyl-(1H,2H,3H,4H,5H,6H)-triazin-2,4-dion,
1,3-Bis-4-chlor-3-trifluormethylphenyl-(1H,2H,3H,4H,5H,6H)-triazin-2,4-dion,
1,3-Bis-3-chlor-4-trifluormethylphenyl-(1H,2H,3H,4H,5H,6H)-triazin-2,4-dion,
1,3-Bis-6-chlorhexyl-(1H,2H,3H,4H,5H,6H)-triazin-2,4-dion,
1,3-Bis-4-phenoxyphenyl-(1H,2H,3H,4H,5H,6H)-triazin-2,4-dion.

Die als Ausgangsmaterial zur Herstellung der erfindungsgemäßen Verbindungen dienenden Triazin-2,4-dione sind zum Teil bekannt (siehe Deutsche Offenlegungsschrift 2 543 497). Neue Verbindungen können analog den dort angegebenen Herstellungsverfahren erhalten werden.

Die Herstellung der erfindungsgemäßen Verbindungen wird unter Verwendung inerter organischer Verdünnungsmittel durchgeführt. Als solche kommen in Frage z. B. Benzol, Toluol, Xylol, Methylenchlorid, Chloroform, Chlorbenzol, Dibutyläther, Dioxan und insbesondere Methanol, Äthanol, Isopropanol, Aceton, Methyläthylketon, Essigsäureäthylester und Acetonitril.

Die Reaktion kann bei Wasserstoffdrücken zwischen 1 und 200 bar, bevorzugt zwischen 20 und 100 bar und Temperaturen zwischen 10°C und 120°C, bevorzugt zwischen 40°C und 100°C durchgeführt werden.

Im erfindungsgemäßen Verfahren verwendbare Katalysatoren sind:

Hydrier-Katalysatoren, die überwiegend Metalle mit den Atomnummern 23 bis 29 in reduzierter und/oder oxidischer Form enthalten. Geeignete Katalysatoren sind beispielsweise Nickel- oder Kobalt-Katalysatoren, wie Nickel-auf-Träger, wobei als Träger anorganische Materialien wie Kieselgur, Kieselsäuren, Aluminiumoxide, Silikate, Aluminiumsilikate, Montmorillonit, Zeolithe, Spinelle, Dolomit, Kaolin, Magnesiumsilikate, Zirkonoxid, Eisenoxid, Zinkoxid, Claciumcarbonat, Siliziumcarbid, Aluminiumphosphat, Borphosphat, Asbest oder Aktiv-Kohle und als organische Katalysatorträger natürlich vorkommende oder synthetische Verbindungen mit hohem Molgewicht wie Seide, Polyamide, Polystyrole, Zellstoff oder Polyurethane verwendbar sind, wobei die Träger in Form von Kugeln, Strängen, Fäden, Zylindern, Polygarnen oder in Pulverform vorliegen können,

Raney-Typ-Katalysatoren, wie Raney-Nickel, W-1-, W-5-, W-6-, W-7-Raney-Nickel wie bei H. Adkins, J. Am. Chem. Soc. 69, 3039 (1974) beschrieben, Raney-Kobalt-Katalysatoren, Raney-Kupfer, Raney-Nickel-Eisen, Raney-Kobalt-Nickel, Raney-Kobalt-Eisen, durch Reduktion von Nickel- oder Kobaltsalzen hergestellte Metallkatalysatoren, wie Urushibara-Nickel oder mit Metallalkylverbindungen, Alkalihydriden, Hydrazin, Boranaten oder Borwasserstoff reduzierte Nickel- oder Kobaltsalze durch Reduktion der Metalloxide oder Metalloxidgemische hergestellte Katalysatoren, die Metalloxide oder- oxidgemische.

Die Katalysatoren können als Beschleuniger eines oder mehrere der folgenden Elemente in Mengen bis zu 10% enthalten:

Li, Na, Ca, Ba, K, Ag, Be, La, Ce, V, Nb, Ta, Mo, W und bis zu 1% der Elemente Ru, Rh, Pd, An, Ir, Pt.

Besonders geeignete Katalysatoren sind:

Raney-Nickel mit 90 Gew.-% Ni und <1 Gew.-% Fe, Ca, Na, Raney-Nickel-Eisen mit 5 bis 30 Gew.-% Fe und <1 Gew.-% Ca, Na und Raney-Kobalt-Eisen mit 10 – 30 Gew.-% Fe.

Der zur Hydrierung notwendige Katalysator wird in Mengen von 10 – 100 Gew.-% bezogen auf zu hydrierende Verbindung eingesetzt.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal

sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonantera z. B. Xenopsylla cheopis, Ceratophyllus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und -Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische

Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z. B. Alkylaryl-polyglykol-äther, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können zur Verstärkung und Ergänzung ihres Wirkungsspektrums je nach beabsichtigter Verwendung mit anderen insektiziden Wirkstoffen kombiniert werden.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Bei hohen Aufwandkonzentrationen weisen die erfindungsgemäßen Verbindungen auch eine gewisse herbizide Wirkung auf.

Bei den folgenden Beispielen zur entwicklungshemmenden Wirkung der Wirkstoffe werden während der gesamten angegebenen Entwicklung der Testtiere die morphologischen Veränderungen, wie zur Hälfte verpuppte Tiere, unvollständig geschlüfte Larven oder Raupen, defekte Flügel, pupale Kutikula bei Imagines etc., als Mißbildungen gewertet. Die Summe der morphologischen Mißbildungen, zusammen mit den während des Häutungsgeschehens oder der Metamorphose abgetöteten Tieren, wird in Prozent der Gesamtzahl der eingesetzten Versuchstiere bestimmt.

## Beispiel A

### Entwicklungshemmende Wirkung/Laphygma-Raupen-Test

| | |
|---|---|
| Testtiere: | Laphygma frugiperda (Raupen) |
| Futter: | 1 cm dicke Scheibe von 3 cm Durchmesser luftangetrocknetes Kunstfutter aus Bohnenkernschrot, Hefe, Vitaminmischung, Blattpulver, Agar und Konservierungsstoff |
| Lösungsmittel: | 10 Gewichtsteile Aceton |
| Emulgator: | 1 Gewichtsteil Alkylarylpolyglykoläther |

Zur Herstellung einer zweckmäßigen Wirkstoffzubereitung vermischt man 2 Gewichtsteile Wirkstoff mit der angegebenen Menge Lösungsmittel, Emulgator und so viel Wasser, daß eine 1%ige Mischung entsteht, die mit Wasser auf die gewünschte Konzentration verdünnt wird.

Jeweils ein Testtier wird auf eine mit 1,5 ml Wirkstoffzubereitung der gewünschten Konzentration angefeuchtete Futterscheibe gesetzt und bis zum Schlüpfen der Imago beobachtet.

Zur Kontrolle wird je ein Testtier auf eine mit 1,5 ml Lösungsmittel- und Emulgator-Wassergemisch der entsprechenden Konzentration angefeuchtete Futterscheibe gesetzt und bis zum Schlüpfen der Imago beobachtet.

Bei diesem Test zeigt z. B. die folgende Verbindung der Herstellungsbeispiele überlegene Wirkung gegenüber dem Stand der Technik: 4

## Beispiel B

### Entwicklungshemmende Wirkung/Fraßtest

| | | |
|---|---|---|
| Testtiere: | Plutella maculipennis | (Raupen im 4. Entwicklungsstadium) |
| Zahl der Testtiere: | | 20 Stück |
| Testtiere: | Phaedon cochleariae | (Larven im 4. Entwicklungsstadium) |
| Zahl der Testtiere: | | 20 Stück |
| Futterpflanzen: | Kohlpflanzen (Brassica olearacea) | |
| Lösungsmittel: | 10 Gewichtsteile Aceton | |
| Emulgator: | 1 Gewichtsteil Alkylarylpolyglykoläther | |

Zur Herstellung einer zweckmäßigen Wirkstoffzubereitung vermischt man 2 Gewichtsteile Wirkstoff mit der angegebenen Menge Lösungsmittel, Emulgator und so viel Wasser, daß eine 1%ige Mischung entsteht, die mit Wasser auf die gewünschte Konzentration verdünnt wird.

Die Testtiere werden mit Blättern der Futterpflanzen, die mit einem gleichmäßigen Spritzbelag der Wirkstoffzubereitung so überzogen werden daß die gewünschte Wirkstoffkonzentration (Wirkstoffmenge pro Flächeneinheit) auf den Blättern erreicht wird, bis zur Entwicklung der Imago gefüttert.

Zur Kontrolle werden nur mit Lösungsmittel- und Emulgator-Wassergemisch der entsprechenden Konzentration überzogene Blätter verfüttert.

Bei diesem Test zeigen z. B. die folgenden Verbindungen der Herstellungsbeispiele überlegene Wirkung gegenüber dem Stand der Technik: 4 und 6

## Beispiel C

### Entwicklungshemmende Wirkung/Kontakttest

| | | |
|---|---|---|
| Testtier: | Dysdercus intermedius | (Larven im 3. Entwicklungsstadium) |
| Zahl der Testtiere: | | 10 Stück |
| Futter: | Samen der Baumwolle (Gossypium hirsutum) | |
| Lösungsmittel: | 10 Gewichtsteile Aceton | |
| Emulgator: | 1 Gewichtsteil Alkylarylpolyglykoläther | |

Zur Herstellung einer zweckmäßigen Wirkstoffzubereitung vermischt man 2 Gewichtsteile Wirkstoff mit der angegebenen Menge Lösungsmittel, Emulgator und so viel Wasser, daß eine 1%ige Mischung entsteht, die mit Wasser auf die gewünschte Konzentration verdünnt wird.

Die Testtiere werden in die Wirkstoffzubereitung der gewünschten Konzentration für 3 Sekunden getaucht und anschließend in Käfigen gehalten und mit unbehandeltem Baumwollsamen und Wasser gefüttert.

Zur Kontrolle werden nur in ein Lösungsmittel- und Emulgator-Wassergemisch der entsprechenden Konzentration getauchte Tiere gehalten und in gleicher Weise gefüttert.

Bei diesem Test zeigt z. B. die folgende Verbindung der Herstellungsbeispiele überlegene Wirkung gegenüber dem Stand der Technik: 1

## Herstellungsbeispiele

Allgemeine Vorschrift:

10 g Ausgangsverbindung und 5 g Hydrierkatalysator werden in 500 ml Methanol gelöst bzw. suspendiert. Diese Lösung bzw. Suspension wird in einem Autoklaven bei P bar und T Grad Celsius N Stunden hydriert. Man läßt dann abkühlen, entspannt und saugt vom Katalysator ab. Das Filtrat wird bis zur Trockne eingeengt. Der zurückbleibende Stoff wird dünnschichtchromatographisch untersucht und anschließend gegebenenfalls aus Chloroform oder Äthanol/Aceton wie 1/1 umkristallisiert.

Tabelle:

| Nr. | Ausgangsverbindung | Katalysator | N (Stdn) | P (bar) | T (°C) | Ausbeute | Produkt | Sch. m. p. |
|---|---|---|---|---|---|---|---|---|
| 1. | 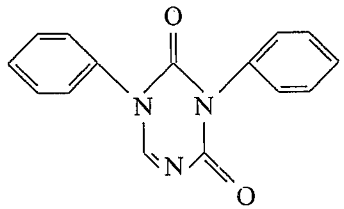 | Raney Ni | 4 | 150 | 100 | 93% | 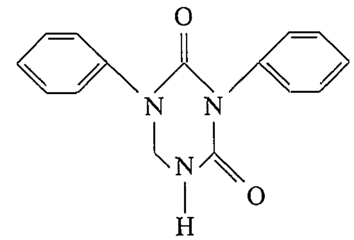 | 218°C |
| 2. | wie 1.) | Raney Ni | 1,5 | 60 | 75 | 91% | wie 1.) | 217°C |
| 3. | wie 1.) | Pd-Kohle | 2 | 65 | 45 | 98% | wie 1.) | 218°C |
| 4. | 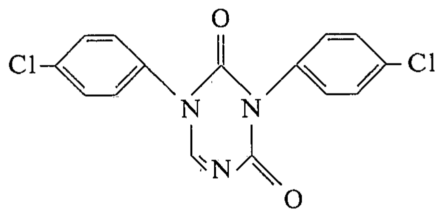 | Raney Ni | 2 | 90 | 95 | 96% | 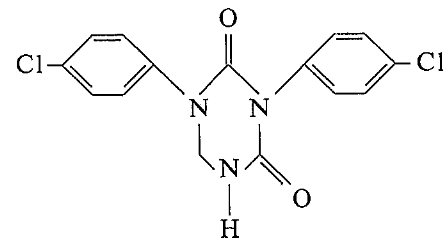 | 173°C |
| 5. | 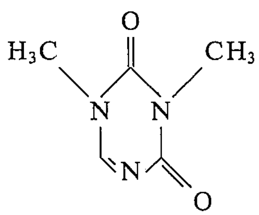 | Raney Ni | 2 | 100 | 80 | 86% | 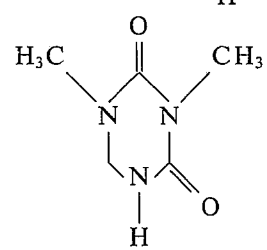 | 169°C |
| 6. | 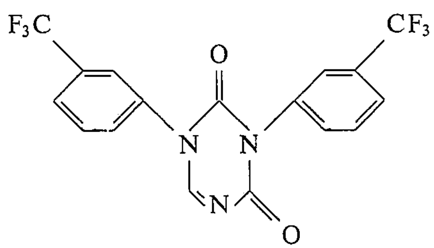 | Raney Ni | 2 | 70 | 70 | 95% | 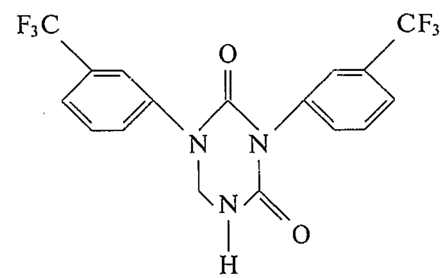 | 143°C |

**Patentansprüche**

1. (1,2,3,4,5,6H)-Triazin-2,4-dione der allgemeinen Formel (I)

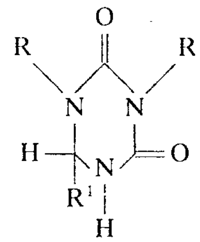

(I)

in welcher

R für gegebenenfalls ein- oder mehrfach durch Halogen, Cyano, Aryloxy, Alkoxy, Halogenalkoxy substituiertes geradkettiges verzweigtes oder cyclisches Alkyl mit bis zu 20 C-Atomen oder für Phenyl oder Naphthyl steht, die gegebenenfalls ein- oder mehrfach durch Halogen, CN, Alkyl $C_{1-4}$, Mono oder Dialkylamino, Halogenalkyl, gegebenenfalls durch Halogen und/oder Halogenalkyl substituiertes Phenoxy, Alkoxy, Halogenalkoxy substituiert sind weiterhin für gegebenenfalls durch Halogen, Cyano, Mono- oder Dialkylamino, Alkoxy substituiertes Phenylsulfonyl oder für gegebenenfalls durch Halogen oder Cyano substituiertes Alkylsulfonyl, steht, und

$R^1$ für Wasserstoff, für geradkettiges verzweigtes oder cyclisches Alkyl mit bis zu 10 C-Atomen das gegebenenfalls substituiert ist durch Halogen, Alkoxy oder Cyano steht.

2. Verfahren zur Herstellung der (1,2,3,4,5,6H)-Triazin-2,4-dione der allgemeinen Formel (I), dadurch gekennzeichnet, daß man (1,2,3,4H)-Triazin-2,4-dione der allgemeinen Formel (II)

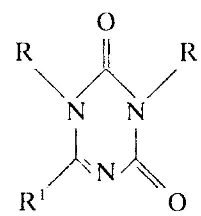

(II)

in welcher
R und $R^1$ die in Anspruch 1 angegebene Bedeutung besitzen,
katalytisch hydriert.

3. Insektizide Mittel gekennzeichnet durch einen Gehalt an mindestens einem (1,2,3,4,5,6H)-Triazin-2,4-dione der allgemeinen Formel (I) gemäß Anspruch 1.

4. Verwendung von (1,2,3,4,5,6H)-Triazin-2,4-dione der allgemeinen Formel (I) gemäß Anspruch 1 zur Bekämpfung von Insekten.

5. Verfahren zur Herstellung insektizider Mittel, dadurch gekennzeichnet, daß man (1,2,3,4,5,6H)-Triazin-2,4-dione der allgemeinen Formel (I) gemäß Anspruch 1 mit Streckmitteln und/oder oberflächenaktiven Stoffen vermischt.

**Claims**

1. (1,2,3,4,5,6H)-Triazine-2,4-diones of the general formula (I)

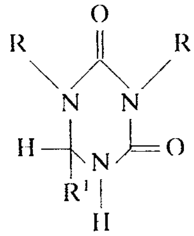

(I)

in which

R represents straight-chain, branched or cyclic alkyl with up to 20 C atoms which is optionally mono-substituted or polysubstituted by hlaogen, cyano, aryloxy, alkoxy or halogenoalkoxy, or represents phenyl or naphthyl which are optionally monosubstituted or polysubstituted by halogen, CN, alkyl-$C_{1-4}$, mono or dialkylamino, halogenoalkyl, phenoxy which is optionally substituted by halogen and/or halogenoalkyl, alkoxy or halogenoalkoxy, and furthermore represents phenylsulphonyl which is optionally substituted by halogen, cyano, mono- or di-alkylamino or alkoxy, or represents alkylsulphonyl which is optionally substituted by halogen or cyano and

$R^1$ represents hydrogen, or represents straihgt-chain, branched or cyclic alkyl with up to 10 C atoms which is optionally substituted by halogen, alkoxy or cyano.

2. Process for the preparation of the (1,2,3,4,5,6H)-triazine-2,4-diones of the general formula (I), characterised in that (1,2,3,4H)-triazine-2,4-diones of the general formula (II)

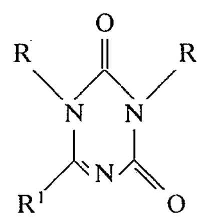

(II)

in which
R and $R^1$ have the meaning indicated in Claim 1,
are catalytically hydrogenated.

3. Insecticidal agents, characterised in that they contain at least one (1,2,3,4,5,6H)-triazine-2,4-dione of the general formula (I) according to Claim 1.

4. Use of (1,2,3,4,5,6H)-triazine-2,4-diones of the general formula (I) according to Claim 1 for combating insects.

5. Process for the preparation of insecticidal agents, characterised in that (1,2,3,4,5,6H)-triazine-2,4-diones of the general formula (I) according to Claim 1 are mixed with extenders and/or surface-active substances.

**Revendications**

1. Des (1,2,3,4,5,6H)-triazine-2,4-diones de formule générale (I):

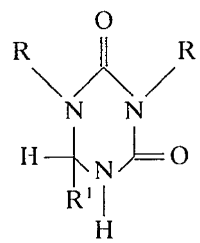

(I)

dans laquelle

R désigne un groupe alkyle à chaîne droite, ramifiée ou cyclique ayant jusqu'à 20 atomes de carbone, éventuellement substitué une ou plusieurs fois par un halogène ou un radical cyano, aryloxy, alkoxy ou halogénalkoxy ou un groupe phényle ou naphthyle, qui sont éventuellement substitués une ou plusieurs fois par un halogène ou un radical CN, alkyle en $C_1$ à $C_4$, mono- ou dialkylamino, halogénalkyle, phénoxy, alkoxy ou halogénalkoxy éventuellement substitués par un halogène et/ou un radical halogénalkyle, ainsi qu'un groupe phénylsulfonyle éventuellement substitué par un halogène ou un radical cyano, mono-alkylamino, dialkylamino ou alkoxy ou un groupe alkylsulfonyle éventuellement substitué par un halogène ou un radical cyano, et

$R^1$ est de l'hydrogène, un groupe alkyle à chaîne droite, à chaîne ramifiée ou cyclique ayant jusqu'à 10 atomes de carbone, qui est éventuellement substitué par un halogène ou un radical alkoxy ou cyano.

2. Procédé de production des (1,2,3,4,5,6H)-triazine-2,4-diones de formule générale (I), caractérisé en ce qu'on hydrogène par voie catalytique des (1,2,3,4H)-triazine-2,4-diones de formule générale (II):

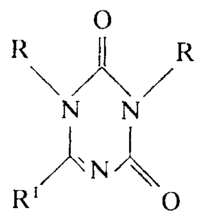

(II)

dan laquelle:

R et $R^1$ ont la définition indiquée dans la revendication 1.

3. Compositions insecticides, caractérisées en ce qu'elles contiennent au moins une (1,2,3,4,5,6H)-triazine-2,4-dione de formule générale (I) suivant la revendication 1.

4. Utilisation de (1,2,3,4,5,6H)-triazine-2,4-diones de formule générale (I) suivant la revendication 1 pour la lutte contre des insectes.

5. Procédé de préparation de compositions insecticides, caractérisé en ce qu'on mélange des (1,2,3,4,5,6H)-triazine-2,4-diones de formule générale (I) suivant la revendication 1 avec des diluants et/ou des agents tensio-actifs.